# EUROPÄISCHE PATENTSCHRIFT

(11) **EP 2 234 506 B1**
(45) Veröffentlichungstag und Bekanntmachung des Hinweises auf die Patenterteilung: **03.07.2013**
(21) Anmeldenummer: 08869101.9
(22) Anmeldetag: 18.12.2008
(51) Int. Cl.: A23L 1/305, A61K 31/198, A61K 8/44

(54) **ZUBEREITUNG UMFASSEND EINE KREATIN-KOMPONENTE, VERFAHREN ZU DEREN HERSTELLUNG UND IHRE VERWENDUNG**
PREPARATION COMPRISING A CREATINE COMPONENT, METHOD FOR THE PRODUCTION THEREOF, AND THE USE THEREOF
PRÉPARATION COMPRENANT UN COMPOSANT DE CRÉATINE, PROCÉDÉ POUR SA FABRICATION, ET SON UTILISATION

(30) Priorität: 21.12.2007 DE 102007062288
(43) Veröffentlichungstag der Anmeldung: 06.10.2010
(73) Patentinhaber: AlzChem AG, 83308 Trostberg (DE)
(72) Erfinder: GASTNER, Thomas, D-84518 Garching an der Alz (DE); Warrikoff, Frauke, D-60596 Frankfurt (DE); NIESS, Barbara, 83308 Trostberg (DE); FUEST, Josef, 83308 Trostberg (DE)
(74) Vertreter: Böhm, Brigitte
(86) Internationale Anmeldenummer: PCT/EP2008/010851
(87) Internationale Veröffentlichungsnummer: WO 2009/083169

(56) Entgegenhaltungen:
- EP-A- 1 051 914
- WO-A-01/89476
- WO-A-2004/073420
- WO-A-2006/015774
- DE-A1-102005 009 990
- US-A- 5 908 864
- US-A1- 2002 131 987
- US-B1- 6 274 161
- US-B1- 6 413 552

## Beschreibung

Gegenstand der vorliegenden Erfindung ist eine Zubereitung umfassend eine Kreatin-Komponente, welche eine ausgezeichnete Bioverfügbarkeit besitzt und zu einer verbesserten Kreatin-Retention im menschlichen und tierischen Körper führt. Ferner betrifft die vorliegende Erfindung ein Verfahren zur Herstellung dieser Zubereitung sowie deren Verwendung als Nahrungsergänzungsmittel, Funktionsnahrungsmittel, Futtermittelzusatz, Arzneimittel und als Zusatz für kosmetische und dermatologische Formulierungen.

Seit Ende der 70iger Jahre des vergangenen Jahrhunderts wird die ergogene Wirkung von Kreatin systematisch untersucht. Bis heute wurden über 300 Studien im Sportbereich durchgeführt, wobei etwa 80 % dieser Studien signifikant positive Effekte des Kreatins auf die Muskelmasse, die Muskelkraft, die fettfreie Körpermasse und die Leistung bei maximaler, kurzzeitiger Muskelanstrengung in verschiedenen Sportarten zeigte. Kreatin-Monohydrat ist heute das wichtigste Nahrungsergänzungsmittel im Sportbereich.

Erst vor kurzer Zeit sind weitere interessante Eigenschaften des Kreatins bekannt geworden. So wurden in zwei Studien signifikant positive Wirkungen einer oralen Kreatin-Supplementation auf die Gehimleistung und die Konzentrationsfähigkeit nachgewiesen (Rae, Caroline et al.: Oral creatine monohydrate supplementation improves brain performance: a double-blind, placebo-controlled, cross-over trial. Proceedings of the Royal Society of London, Series B: Biological Sciences (2003), 270(1529), 2147-2150; Watanabe, Airi et al.: Effects of creatine on mental fatigue and cerebral hemoglobin oxygenation. Neuroscience Research (Oxford, United Kingdom) (2002), 42(4), 279-285).

Weiterhin konnte gezeigt werden, dass Kreatin antioxidative und neuroprotektive Eigenschaften besitzt und somit auch zur Vorbeugung von Schädigungen der Zellen durch Umwelteinflüsse eingesetzt werden kann (Sestili, Piero et al.: Creatine supplementation affords cytoprotection in oxidatively injured cultured mammalian cells via direct antioxidant activity. Free Radical Biology & Medicine (2006), 40(5), 837-849; P. Klivenyi et al.: Neuroprotective effects of creatine in a transgenic animal model of amyotrophic lateral sclerosis. Nature Medicine 5, 347-350 (1999)). Kreatin wird deshalb in Zukunft auch im Anti-Aging Bereich stark an Bedeutung gewinnen.

Die positiven Effekte von Kreatin werden derzeit auch im medizinischen Bereich intensiv untersucht, wobei sich Kreatin bei der Behandlung von Parkinson und der Amyotrophen Lateralen Sklerose (ALS) in der klinischen Phase 3 und bei Corea-Huntington in Phase 2 befindet (EP 804 183 B1). Auch von einem erfolgreichen Einsatz von Kreatin als Therapeutikum gegen Asthma wurde bereits berichtet (EP 911 026 B1). Beim Aufbau von Knochen zeigte Kreatin sowohl in vitro als auch in vivo positive Effekte. Der Einsatz zur Stärkung der Knochen und zur Behandlung und Vorbeugung von degenerativen Knochen- und Knorpel-Erkrankungen wie etwa Osteoporose wurde untersucht und lieferte sehr positive Ergebnisse (EP 1 100 488 B1; Gerber, I et al.: Stimulatory effects of creatine on metabolic activity, differentiation and mineralization of primary osteoblast-like cells in monolayer and micromass cell cultures. European Cells and Materials (2005), 10, 8-22; Chilibeck, P. D. et al.: Creatine monohydrate and resistance training increase bone mineral content and density in older men. Journal of Nutrition, Health & Aging (2005), 9(5), 352-355).

Weiterhin ist bekannt, dass eine Kreatin-Supplementierung zu einer Erhöhung der Körpermasse führt. Dies ist zu Anfang auf eine vermehrte Aufnahme von Wasser in den Muskel zurückzuführen. Langfristig gesehen führt Kreatin aber indirekt durch vermehrte Proteinsynthese oder einen verminderten Proteinkatabolismus in den Myofibrillen zu einer Erhöhung der Muskelmasse (Int J Sports Med 21 (2000), 139-145). Als Ergebnis erhält man somit eine erhöhte fettfreie Körpermasse.

Neben dem Kreatin selbst, also dem Kreatin-Monohydrat, haben sich zwischenzeitlich aber auch zahlreiche Kreatin-Salze, wie das Kreatinascorbat, -citrat, -pyruvat und andere, ebenfalls als geeignete Nahrungsergänzungsmittel erwiesen. Stellvertretend seien an dieser Stelle das europäische Patent EP 894 083 und die deutsche Offenlegungsschrift DE 197 07 694 A1 als Stand der Technik genannt.

Der Metabolismus und die Wirkungsweise von Kreatin sind sehr gut untersucht. Die Biosynthese geht von Glycin und L-Arginin aus. Bei Säugetieren wird vor allem in den Nieren, aber auch in der Leber und der Bauchspeicheldrüse, die Guanidino-Gruppe des L-Arginins durch das Enzym Aminotransferase gespalten und eine N-C-N-Gruppe auf das Glycin übertragen. Das L-Arginin wird hierbei in L-Ornithin umgewandelt. Die so gebildete Guanidinoessigsäure wird im nächsten Schritt, der bei Vertebraten vor allem in der Leber erfolgt, mit Hilfe des Enzyms Transmethylase in Kreatin umgewandelt. Hierbei dient das S-Adenosylmethionin als Methylgruppen-Donor. Das Kreatin diffundiert anschließend in den Blutkreislauf und wird so zu den Zielorganen transportiert. Der Transport durch die Zellmembran in die Zellen geschieht hierbei durch einen spezifischen NaCl-abhängigen Kreatin-Transporter (Speer O, Neukomm LJ, Murphy RM, Zanolla E, Schlattner U, Henry H, Snow RJ, Wallimann T. Creatine transporters: a reappraisal. Mol Cell Biochem. 2004 Jan-Feb; 256-257(1-2):407-24).

Kreatin spielt im Energiestoffwechsel der Zelle eine wichtige Rolle, wobei es als energiereiches Phosphokreatin neben dem Adenosintriphosphat (ATP) eine wesentliche Energiereserve des Muskels darstellt. Im Ruhezustand des Muskels kann ATP eine Phosphat-Gruppe auf Kreatin übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion, durch das Enzym Kreatinkinase eine Phosphatgruppe auf Adenosindiphosphat übertragen und somit ATP zurückbilden. Dies wird auch als Lohmann-Reaktion bezeichnet.

Weiterhin besitzt Kreatin eine wichtige Funktion bei der Übertragung von Energie in der Zelle. Das so genannte Kreatin-Shuttle-System transportiert Energie von den Mitochondrien an die Stellen in der Zelle, an denen die Energie benötigt wird.

Bei starker und über längere Zeit anhaltender Muskelarbeit sind die natürlich im Körper vorhandenen Kreatin-Vorräte rasch erschöpft. Aus diesem Grund haben sich insbesondere bei Leistungssportlern gezielte Kreatin-Gaben positiv auf die Ausdauer und Leistungsfähigkeit ausgewirkt, wobei unerwünschte Anreicherungsprozesse im Körper oder nachteilige Abbauprodukte unbekannt sind. Der Grund hierfür ist darin zu sehen, dass Kreatin bei einer übermäßigen Zufuhr vom Körper über die Nieren ausgeschieden wird. Weiterhin wandelt sich Kreatin mit einer konstanten Rate in das zyklische Abbauprodukt Kreatinin um, welches ebenfalls über die Nieren ausgeschieden wird und somit einen zweiten metabolischen Abbauweg darstellt.

Die Aufnahme von Kreatin aus den Darm und der Transport in die Muskulatur wird von einem NaCl-abhängigen Kreatin-Transporter gesteuert und kann durch die gleichzeitige Aufnahme von Kohlenhydraten und Proteinen positiv beeinflusst werden. Dabei zeigte sich, dass die Kombination von Kreatin und Kohlenhydraten im Vergleich zur alleinigen Einnahme von Kreatin zu einem um 60 % höheren Anstieg des Kreatin-Gehalts im Muskel führen kann (Green AL, Hultman E, Macdonald IA, Sewell DA, Greenhaff PL. Carbohydrate ingestion augments skeletal muscle creatine accumulation during creatine supplementation in humans. Am J Physiol. 1996 Nov; 271 (5 Pt 1):E821-6). Es konnte gezeigt werden, dass die Ausschüttung von Insulin bei der Aufnahme von Kreatin in die Muskelzellen eine wichtige Rolle spielt. Zwischen der Erhöhung der Kreatin-Konzentration in der Muskulatur und der ausgeschütteten Menge an Insulin besteht ein linearer Zusammenhang (Steenge GR, Simpson EJ, Greenhaff PL. Proteinand carbohydrate-induced augmentation of whole body creatine retention in humans. J Appl Physiol. 2000 Sep; 89(3):1165-71).

Zur Verbesserung der Aufnahme von Kreatin in den Körper wurden weitere Formulierungen vorgeschlagen. So beschreibt die Patentanmeldung DE 10 2006 050 931.5 eine feste oder wässrige alkalische Zubereitung umfassend eine Kreatin-Komponente, welche ein Puffersystem enthält, das einen pH-Wert von 8,0 bis 12,0 einstellt.

US 6 274 161 besdreibt Kreatini-Monohydrat in (Citrat) puffersystemen mit pH 2.5-6.5.

US 2003215506 beansprucht eine den Kreatin-Transport verbessernde Formulierung, welche IGF-1 modulierende Substanzen enthält, insbesondere Proteine, Colostrum oder rekombinantes IGF-1.

Neben seinen unbestrittenen positiven physiologischen Eigenschaften besitzt Kreatin aber auch den Nachteil, dass es in den entsprechenden wässrigen Lösungen keine ausgeprägte Stabilität besitzt. Kreatin zyklisiert hierbei durch die Abspaltung von Wasser zu Kreatinin. Die Zyklisierungsgeschwindigkeit ist vom pH-Wert der Lösung und der Temperatur abhängig, wobei die Konzentration keine Rolle spielt. Besonders im sauren pH-Bereich zwischen 3 und 4 verläuft die Umwandlung in Kreatinin schnell. Aufgrund des schnellen Abbaus von Kreatin in diesem Milieu ist der Einsatz in wässrigen oder feuchten Formulierungen für die menschliche und tierische Ernährung praktisch ausgeschlossen. Bereits der pH-Wert des Magens von 1 bis 2 kann je nach Verweilzeit zu einem deutlichen Abbau des Kreatins zu Kreatinin führen. (Greenhaff, P.L.: Factors Modifying Creatine Accumulation in Human Skeletal Muscle. In: Creatine.

From Basic Science to Clinical Application. Medical Science Symposia Series Volume 14, 2000, 75-82).

Aus den bzgl. der Aufnahme aus dem Darm und dem Transport in das Zielgewebe sowie bzgl. der Stabilität des Kreatins geschilderten Nachteilen des Standes der Technik hat sich für die vorliegende Erfindung die Aufgabe gestellt, Zubereitungen zu entwickeln, welche das Kreatin besser vor dem Abbau zu Kreatinin im Magen schützen und anschließend zu einer verbesserten Aufnahme aus dem Darm führen. Entscheidend ist aber insbesondere eine optimale Aufnahme und somit Retention des Kreatins im Zielgewebe. Eine weitere Aufgabe war somit, dass das aus dem Darm absorbierte Kreatin optimal im Zielgewebe aufgenommen wird und nicht als solches über die Nieren ausgeschieden oder in Kreatinin umgewandelt wird, welches für den Körper nutzlos ist und ebenfalls über die Nieren aus dem Körper ausgeschieden werden muss. Weiterhin sollten die Zubereitungen gute organoleptische Eigenschaften besitzen. Die erfindungsgemäßen Zubereitungen sollten ferner eine verbesserte Bioverfügbarkeit in dermatologischen und kosmetischen Zubereitungen besitzen und somit zu einer verbesserten Aufnahme der Kreatin-Komponente in die Haut beitragen.

Gelöst wurde die Aufgabe durch die Bereitstellung einer Zubereitung umfassend eine Kreatin-Komponente mit 30-80 Gew.-% bezogen auf das Gesamtgewicht der Zubereitung und ein Puffersystem, wobei das Puffersystem aus mindestens einem Natriumsalz einer organischen Säure besteht, und in einer Menge von 0,1 bis 90,0 Gew.-%, bezogen auf das Gesamtgewicht der Zubereitung, vorliegt und wobei die Zubereitung einen pH-Wert von 2,5 bis 7,9 aufweist, wobei das molare Verhältnis von Kreatin-Komponente zu Natrium-Ionen zwischen 1 : 1 bis 10 liegt.

Es konnte gezeigt werden, dass mit diesen Zubereitungen die Aufgabenstellung voll erfüllt werden konnte, nämlich, dass Kreatin durch das Puffersystem im Magen besser vor einer Umwandlung in Kreatinin geschützt wird. Überraschend hat sich herausgestellt, dass die neuen Formulierungen zu einer deutlich besseren Aufnahme aus dem Darm führen, eine deutlich höhere Bioverfügbarkeit aufweisen und somit besser in das Zielgewebe aufgenommen werden. Außerdem besitzt die erfindungsgemäße Zubereitung sehr gute organoleptische Eigenschaften.

Bei der Kreatin-Komponente handelt es sich vorzugsweise um Kreatin, Kreatin-Monohydrat, Guanidinoessigsäure, Kreatin-Ester, Creatinol, Creatinol-O-phosphat bzw. Mischungen aus mindestens zwei dieser Verbindungen davon. Guanidinoessigsäure, Kreatin-Ester, Creatinol und Creatinol-O-phosphat werden bekannterweise im Körper in Kreatin umgewandelt und können auf diese Weise ihre Wirkung entfalten. Als Kreatin-Ester können insbesondere Kreatin-Methylester und Kreatin-Ethylester eingesetzt werden. Als besonders bevorzugte Kreatin-Komponenten im Sinne der Erfindung sind Kreatin-Monohydrat und Guanidinoessigsäure anzusehen.

In einer weiteren bevorzugten Ausführungsform ist die Kreatin-Komponente ein Salz, eine Anlagerungs- oder Komplexverbindung dieser Komponente, vorzugsweise mit Äpfelsäure, Ascorbinsäure, Bernsteinsäure, Brenztraubensäure, Fumarsäure, Asparaginsäure, Adipinsäure, Gluconsäure, α-Ketoglutarsäure, Weinsäure, α-Liponsäure, Oxalsäure, Pyroglutaminsäure, 3-Nicotinsäure, Maleinsäure, Schwefelsäure, Essigsäure, Ameisensäure, Phosphorsäure, Salzsäure, 2-Hydroxybenzoesäure, L-Camitin, Acetyl-L-Carnitin, Taurin, Betain, Cholin und Methionin.

Bezüglich des vom Puffersystem umfassten Natriumsalzes einer organischen Säure haben sich als organische Säuren insbesondere Äpfelsäure, Ascorbinsäure, Bernsteinsäure, Brenztraubensäure, Fumarsäure, Adipinsäure, Asparaginsäure, Gluconsäure, α-Ketoglutarsäure, α-Liponsäure, Weinsäure, Oxalsäure, Pyroglutaminsäure, 3-Nicotinsäure, Zitronensäure, Maleinsäure, Essigsäure, Ameisensäure, 2-Hydroxybenzoesäure, Sorbinsäure und Isoascorbinsäure als besonders geeignet erwiesen.

Es ist als erfindungswesentlich anzusehen, dass das Puffersystem einen pH-Wert von 2,5 bis 7,9 und vorzugsweise 5,0 bis 7,0 einstellt. Als bevorzugtes Puffersystem sieht die vorliegende Erfindung eine Mischung aus Trinatriumcitrat und/oder Dinatriumcitrat und/oder Mononatriumcitrat vor, wobei eine Mischung aus Trinatriumcitrat und Dinatriumcitrat besonders bevorzugt ist. Das Verhältnis von Trinatriumcitrat und Dinatriumcitrat kann in weiten Bereichen frei gewählt werden, wobei dieses so gewählt wird, dass der pH-Wert der Formulierung sich auf 2,5 bis 7,9, bevorzugt auf 5,0 bis 7,0 einstellt. Hierbei ist es von Vorteil, dass bei der richtigen Wahl des Mischungsverhältnisses die Einsatzmenge praktisch nicht eingeschränkt wird. So stellt sich bei der Verwendung einer 1 : 1 Mischung zwangsläufig ein pH-Wert von 6,4 ein, wobei dieser unabhängig von der eingesetzten Gesamtmenge an Puffer ist.

Somit lässt sich ein aus organoleptischer Sicht akzeptabler pH-Wert einstellen und gleichzeitig wird das Kreatin überraschend gut vor dem Einfluss von Säuren, insbesondere der Magensäure geschützt, wodurch eine Umwandlung in Kreatinin vermieden wird. Die hervorragende Wirkung des Puffersystems, welche einen Abbau von Kreatin in Kreatinin weitgehend verhindert, war im beanspruchten pH-Bereich in keiner Weise vorhersehbar.

Die Formulierung ist bzgl. der Puffer-Komponente nicht limitiert, wobei insbesondere die Menge der Puffer-Komponente, in denen sie in der Zubereitung vorliegen kann, keine Einschränkung darstellt. Aus ernährungsphysiologischen Gründen werden allerdings Mengen empfohlen, die zwischen 0,1 und 90,0 Gew.-%, bezogen auf das Gesamtgewicht der Zubereitung, liegen. Besonders bevorzugt sind Mengen zwischen 2,5 und 50,0 Gew.-% und insbesondere 15,0 bis 40,0 Gew.-%, bezogen auf das Gesamtgewicht der Zubereitung.

Überraschend hat sich bei der Verwendung der beschriebenen Puffersysteme herausgestellt, dass diese nicht nur zu einem geringeren Abbau des Kreatins im Magen führen, sondern dass das verabreichte Kreatin auch besser in die Zellen aufgenommen wird. So konnte in einem Experiment gezeigt werden, dass die erfindungsgemäßen Formulierungen zu einem deutlich höheren Anstieg der Kreatin-Konzentrationen im Muskel führen, als dies bei der Verwendung von Kreatin-Monohydrat der Fall ist.

In diesem Zusammenhang ergab sich überraschenderweise, dass der Natrium-Gehalt der Formulierung einen entscheidenden Einfluss auf die Bioverfügbarkeit und die Aufnahme von Kreatin in die Zellen hat. Die Verwendung einer Mischung aus Kreatin und Natriumsalzen zur Verbesserung der Aufnahme von Kreatin in den Muskel wurde bisher noch nicht beschrieben und bietet gegenüber der bisherigen Praxis der Verwendung von hohen Kohlenhydrat- oder Protein-Dosen deutliche Vorteile. Hierbei erwies sich ein molares Verhältnis von Kreatin-Komponente zu Natrium-Ionen zwischen 1 : 1 bis 10, insbesondere zwischen 1 : 1 bis 3 und besonders bevorzugt zwischen 1 : 2 bis 3 als vorteilhaft.

Die vorliegende Erfindung sieht somit neben dem Puffersystem auch optional die Einarbeitung von mindestens einem weiteren Natriumsalz, welches bevorzugt physiologisch akzeptabel ist, in die Kreatin-Zubereitungen vor. Hierfür kommen beispielsweise Natriumchlorid, Natriumsulfat, Natriumacetat, Natriumadipat, Natriumcitrat, Natriumgluconat, Natriumascorbat, Natriumpantothenat, Natriumtartrat und Natriumlactat oder Mischungen dieser Salze in Frage.

Der Anteil dieser Natriumsalze ist relativ unkritisch, doch hat es sich als besonders vorteilhaft erwiesen, diese weiteren Natriumsalze in einer Menge von 0,1 bis 75,0 Gew.-%, insbesondere 1,0 bis 55,0 Gew.-% und besonders bevorzugt 5,0 bis 10,0 Gew.-%, bezogen auf das Gesamtgewicht der Zubereitung, einzusetzen.

Die Verwendung der beschrieben Puffersysteme erscheint somit ideal, da zum einen die Stabilität des Kreatins gegenüber Säuren erhöht und somit der Abbau von Kreatin im Magen vermieden wird. Weiterhin verbessern die enthaltenen Natrium-Ionen die Aufnahme in die Zellen, wobei dieser Effekt auch über die Zugabe von weiteren Natriumsalzen noch verstärkt werden kann.

Gemäß einer bevorzugten Ausführungsform enthält die erfindungsgemäße Zubereitung noch weitere physiologisch aktive Verbindungen, wie z. B. Kohlenhydrate, Fette, Aminosäuren, Proteine, Vitamine, Mineralstoffe, Spurenelemente sowie deren Derivate und Mischungen davon. Besonders bevorzugt sind die zusätzlichen physiologisch aktiven Verbindungen, ausgewählt aus der Gruppe Kohlenhydrate, gesättigte oder ungesättigte Fettsäuren, gesättigte oder ungesättigte Triglyceride, Aminosäuren, Aminosäure-Derivate wie Taurin, Carnitin, Glutathion oder Proteine wie z.B. Gelatine, Vitamine, Vitaminoide wie z.B. Ubichinone, α-Liponsäure, Inositol, Phospholipide, Mineralstoffe und Spurenelemente sowie Mischungen davon. Der Zusatz von α-Liponsäure zur Zubereitung dient der Verbesserung der Bioverfügbarkeit.

Ein weiterer Gegenstand der vorliegenden Erfindung ist ein Verfahren zur Herstellung der erfindungsgemäßen Zubereitung, wobei die Kreatin-Komponente, bevorzugt als Pulver, vorgelegt wird, mindestens ein Puffersystem, bestehend aus einer Mischung einer schwachen Säure und der korrespondierenden Base, eingearbeitet wird und, ggf. weitere Natriumsalze, physiologisch aktive Verbindungen und/oder α-Liponsäure zugegeben werden. In einer bevorzugten Ausführungsform erfolgt die Einarbeitung homogen. Ferner ist bevorzugt, dass die schwache Säure eine organische Säure ist, und dass die korrespondierende Base ein Natriumsalz der schwachen Säure ist.

Die vorliegende Erfindung beansprucht des Weiteren die Verwendung der erfindungsgemäßen Kreatin-Zubereitung als Nahrungsergänzungsmittel, Funktionsnahrungsmittel, Futtermittelzusatz und/oder Arzneimittel.

Als Applikationsformen haben sich Pulver, Granulate, Pastillen, Kapseln, Tabletten, Brausetabletten, Lösungen, Säfte und/oder Gelee-Produkte als besonders geeignet erwiesen. Dabei kann es in Abhängigkeit vom jeweiligen konkreten Verwendungsfall durchaus empfehlenswert sein, die Kreatin-Zubereitung in Kombination mit anderen physiologisch aktiven Wirkstoffen einzusetzen.

Die beschriebenen positiven Effekte entfalten die erfindungsgemäßen Zubereitungen auch bei Tieren, so dass der Einsatz auch in diesem Bereich vorgesehen wird. Werden die beschriebenen Kreatin-Formulierungen als Futtermittelzusatz verwendet, ist insbesondere die Verabreichung an Zucht - und Masttiere sowie Tiere im Leistungssport als bevorzugt anzusehen und in diesem Zusammenhang besonders bevorzugt an Schweine, Pferde, Geflügel und Fische, wobei sich die Verwendung als Ersatzmittel für Tier - und/oder Fischmehl sowie daraus hergestellte Produkte als besonders geeignet erwiesen hat. Der Ersatz kann dabei als Teil- oder auch Vollersatz stattfinden.

Weiterhin können die neuen Kreatin-Zubereitungen auch als Nahrungsergänzung oder Ernährungsbestandteil für Haustiere, wie Hunde, Katzen und Vögel, eingesetzt werden.

Es hat sich als besonders vorteilhaft erwiesen, wenn die Zubereitungen die Kreatin-Komponente in Tagesdosen von 5 mg bis 400 mg, bevorzugt 10 bis 250 mg und besonders bevorzugt 30 mg bis 100 mg pro kg Körpergewicht enthalten.

Der Anteil an der gesamten Zubereitung kann in weiten Bereichen variieren. Die Kreatin-Komponente kann in Mengen von 5 bis 90,0 Gew.-%, insbesondere 30 bis 80,0 Gew.-% und besonders bevorzugt 40,0 bis 60,0 Gew.-%, bezogen auf das Gesamtgewicht der Zubereitung, eingesetzt werden. Die Tagesdosen können als Einzeldosis oder in mehreren Dosen über den Tag verteilt aufgenommen werden. Es hat sich als vorteilhaft erwiesen, die Zubereitung auf mindestens zwei Einzeldosen über den Tag zu verteilen.

Entsprechend den für Kreatin bekannten Verwendungsgebieten können die Zubereitungen im Rahmen der vorliegenden Erfindung auch in kosmetischen oder dermatologischen Zubereitungen verwendet werden. Hierbei ergibt sich durch das Puffersystem und die Natrium-Ionen ein deutlich verbessertes Aufnahmeprofil in die Haut. Es hat sich als vorteilhaft erwiesen, den kosmetischen oder dermatologischen Zubereitungen neben dem Puffersystem zusätzlich eine Quelle für Chlorid-Ionen, insbesondere Natriumchlorid, zuzusetzen. Im Gegensatz zur oralen Aufnahme der Zubereitung, bei der die Chlorid-Ionen durch die Magensäure bereitgestellt werden, wird hierdurch die Aufnahme in die Haut optimiert. Als bevorzugt sind Zubereitungen anzusehen, die in Form von Cremes, Lotionen, Sprays, Mousse, wässrigen oder wässrig-ethanolischen Lösungen, Tränkungsmedien für Tücher, wasserfreien oder wasserhaltigen Stiften oder Mikroemulsionen vorliegen. Als ganz besonders bevorzugt ist der topische Anwendungsbereich anzusehen.

Insgesamt stellen die vorgeschlagenen Zubereitungen und ihre Verwendung eine weitere Fortentwicklung des Standes der Technik hinsichtlich einer Erhöhung der Stabilität von Kreatin-Zubereitungen und einer wesentlichen Verbesserung der Bioverfügbarkeit dar, wobei die Zubereitungen sich weiterhin durch gute organoleptische Eigenschaften auszeichnen.

Hierbei wird sowohl die Verwendung als Nahrungsergänzungsmittel, Funktionsnahrungsmittel und Futtermittel als auch in dermatologischen und kosmetischen Formulierungen vorgesehen.

Die nachfolgenden Beispiele veranschaulichen die Vorteile der vorliegenden Erfindung.

### Beispiele

### 1. Nahrungsergänzungsmittel

Nachfolgend sind typische Zusammensetzungen aufgeführt, deren Bestandteile bei Raumtemperatur in 500 ml Fruchtsaft, Wasser, Joghurt und/oder Molke eingebracht werden.

| | | |
|---|---|---|
| 1.1 | 3.000 mg | Kreatin Monohydrat |
| | 950 mg | Trinatriumcitrat |
| | 1050 mg | Dinatriumcitrat |
| | | |
| 1.2 | 1000 mg | Kreatin Monohydrat |
| | 1430 mg | Natriumgluconat |
| | 1200 mg | Gluconsäure |
| | | |
| 1.3 | 1500 mg | Kreatin Monohydrat |
| | 1800 mg | Natriummalat |
| | 1000 mg | Natriumhydrogenmalat |
| | 500 mg | Guanidinoessigsäure |
| | 500 mg | Betain |
| | 300 mg | α-Liponsäure |
| | 400 mg | (MgCO₃)₄·Mg(OH)₂·5H₂O = ca. 100 Mg |
| | | |
| 1.4 | 1.500 mg | Kreatin Monohydrat |
| | 900 mg | Natriumsuccinat |
| | 600 mg | Natriumhydrogensuccinat |
| | 1.000 mg | Glucosamin |
| | 300 mg | Chondroitinsulfat |
| | 500 mg | Methionin |

### 2. Futtermittel

2.1 Eine Formulierung bestehend aus 2 kg Kreatin Monohydrat, 5 kg Inulin, 600 g Dinatriumcitrat und 655 g Trinatriumcitrat wurde in 100 kg einer typischen Rezeptur für Futterpellets zur Futterergänzung für Pferde eingebracht.
2.2 Als Masterbatch wurde in 500 g eines handelsüblichen Dosenfutters für Katzen homogen folgende Formulierung eingebracht: 3.000 mg Creatinolsulfat, 3.000 mg Kreatin Monohydrat, 40 mg Magnesiumstearat, 25 mg Carboxymethylcellulose und 135 mg Lactose, 500 mg Gluconsäure und 1000 mg Natriumgluconat.

### 3. Zubereitungen für kosmetische Cremes

3.1 In eine handelsübliche Wasser-in-Öl-Basiscreme wurden 0,6 % Kreatin Monohydrat, 0,19 % Dinatriumcitrat, 0,21 % Trinatriumcitrat und 0,2 % Natriumchlorid homogen eingebracht.
Die Creme eignet sich u. a. zur Behandlung von sensitiven, defizitären und hypoaktiven Hautzuständen, sowie gegen vorzeitige Hautalterung und umweltbedingte negative Veränderungen der Haut.

### 4. Bioverfügbarkeit

Drei Probanden-Gruppen von jeweils zwanzig Personen wurden so zusammengestellt, dass in allen Gruppen etwa die gleichen durchschnittlichen Ausgangswerte von Kreatin in der Muskeltrockenmasse vorhanden waren.

Über sechs Wochen wurde den drei Gruppen täglich eine erfindungsgemäße Zubereitung nach Beispiel 1.1, Kreatin Monohydrat oder Kreatin Monohydrat und 75 g Glucose verabreicht. Die Dosis wurde dabei so gewählt, dass von jedem Proband pro Tag jeweils 3,0 g reines Kreatin Monohydrat aufgenommen wurde. Unmittelbar vor der Studie und sechs Wochen nach der Einnahme wurde der Kreatin-Gehalt im Muskel mittels Muskelbiopsie gemessen. Die Ergebnisse sind in Abbindung 1 dargestellt.

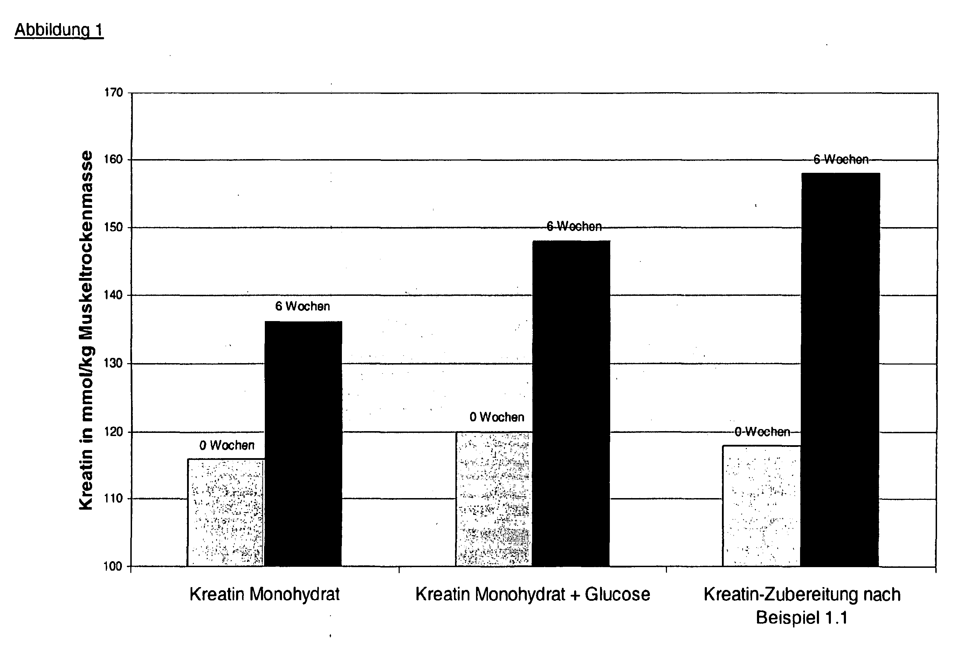

## Patentansprüche

1. Zubereitung umfassend eine Kreatin-Komponente mit 30-80 Gew.-% bezogen auf das Gesamtgewicht der Zubereitung und ein Puffersystem, wobei das Puffersystem aus mindestens einem Natriumsalz einer organischen Säure besteht, und in einer Menge von 0,1 bis 90,0 Gew.-%, bezogen auf das Gesamtgewicht der Zubereitung, vorliegt und wobei die Zubereitung einen pH-Wert von 2,5 bis 7,9 aufweist, wobei das molare Verhältnis von Kreatin-Komponente zu Natrium-Ionen zwischen 1 : 1 bis 10 liegt.

2. Zubereitung nach Anspruch 1, **dadurch gekennzeichnet, dass** die organische Säure ausgewählt ist aus Äpfelsäure, Ascorbinsäure, Bernsteinsäure, Brenztraubensäure, Fumarsäure, Adipinsäure, Asparaginsäure, Gluconsäure, α-Ketoglutarsäure, α-Liponsäure, Weinsäure, Oxalsäure, Pyroglutaminsäure, 3-Nicotinsäure, Zitronensäure, Maleinsäure, Essigsäure, Ameisensäure, 2-Hydroxybenzoesäure, Sorbinsäure und Isoascorbinsäure.

3. Zubereitung nach einem der vorhergehenden Ansprüche, **dadurch gekennzeichnet, dass** es sich bei dem Puffersystem um Trinatriumcitrat, Dinatriumcitrat und/oder Mononatriumcitrat handelt.

4. Zubereitung nach einem der vorhergehenden Ansprüche, **dadurch gekennzeichnet, dass** es sich bei dem Puffersystem um Trinatriumcitrat und Dinatriumcitrat handelt.

5. Zubereitung nach einem der vorhergehenden Ansprüche, **dadurch gekennzeichnet, dass** es sich bei der Kreatin-Komponente um Kreatin, Kreatin-Monohydrat, Guanidinoessigsäure, Kreatin-Ester, Creatinol oder Creatinol-O-phosphat oder einer Mischung aus mindestens zwei dieser Verbindungen handelt.

6. Zubereitung nach einem der vorhergehenden Ansprüche, **dadurch gekennzeichnet, dass** es sich bei der Kreatin-Komponente um mindestens ein Salz, mindestens eine Anlagerungs- und/oder mindestens eine Komplexverbindung der Kreatin-Komponente handelt.

7. Zubereitung nach Anspruch 5, **dadurch gekennzeichnet, dass** das mindestens eine Salz, die mindestens eine Anlagerungs- und/oder die mindestens eine Komplexverbindung der Kreatin-Komponente mit Äpfelsäure, Ascorbinsäure, Bernsteinsäure, Brenztraubensäure, Fumarsäure, Asparaginsäure, Adipinsäure, Gluconsäure, α-Ketoglutarsäure, Weinsäure, α-Liponsäure, Oxalsäure, Pyroglutaminsäure, 3-Nicotinsäure, Maleinsäure, Schwefelsäure, Essigsäure, Ameisensäure, Phosphorsäure, Salzsäure, 2-Hydroxybenzoesäure, L-Camitin, Acetyl-L-Carnitin, Taurin, Betain, Cholin und/oder Methionin gebildet ist.

8. Zubereitung nach einem der vorhergehenden Ansprüche, **dadurch gekennzeichnet, dass** die Kreatin-Komponente zur Verabreichung in Tagesdosen von 5 mg bis 400 mg pro kg Körpergewicht vorgesehen ist.

9. Zubereitung nach einem der vorhergehenden Ansprüche, **dadurch gekennzeichnet, dass** die Zubereitung zusätzlich physiologisch aktive Verbindungen, ausgewählt aus der Gruppe Kohlenhydrate, gesättigte oder ungesättigte Fettsäuren, gesättigte oder ungesättigte Triglyceride, Aminosäuren, Aminosäure-Derivate wie Taurin, Carnitin, Glutathion oder Proteine wie z.B. Gelatine, Vitamine, Vitaminoide wie z.B. Ubichinone, α-Liponsäure, Inositol, Phospholipide, Mineralstoffe und Spurenelemente sowie Mischungen davon, enthält.

10. Zubereitung nach einem der vorhergehenden Ansprüche, **dadurch gekennzeichnet, dass** die Zubereitung zusätzlich ein Natriumsalz oder ein Gemisch davon und/oder α-Liponsäure enthält.

11. Zubereitung nach Anspruch 10, **dadurch gekennzeichnet, dass** das Natriumsalz oder das Gemisch davon ausgewählt ist aus Natriumchlorid, Natriumsulfat, Natriumacetat, Natriumadipat, Natriumcitrat, Natriumgluconat, Natriumascorbat, Natriumpantothenat, Natriumtartrat und Natriumlactat.

12. Zubereitung nach einem der Ansprüche 10 oder 11, **dadurch gekennzeichnet, dass** das Natriumsalz in einer Menge von 0,1 bis 75,0 Gew.-%, bezogen auf das Gesamtgewicht der Zubereitung, vorliegt.

13. Zubereitung nach einem der vorhergehenden Ansprüche, **dadurch gekennzeichnet, dass** die Zubereitung als Pulver, Granulat, Pastille, Kapsel, Tablette, Brausetablette, Lösung, Saft und/oder Gelee-Produkt vorliegt.

14. Verwendung der Zubereitung nach einem der vorhergehenden Ansprüche als Nahrungsergänzungsmittel, Funktionsnahrungsmittel, Futtermittelzusatz und/oder als Zusatz für kosmetische und/oder dermatologische Formulierungen, insbesondere im topischen Anwendungsbereich.

15. Verwendung nach Anspruch 14, **dadurch gekennzeichnet, dass** die kosmetische und/oder dermatologische Formulierung eine Creme, eine Lotion, ein Spray, ein Mousse, eine wässrige oder wässrig-ethanolische Lösung, ein Tränkungsmedium für Tücher, ein wasserfreier oder wasserhaltiger Stift oder eine Mikroemulsion ist.

16. Arzneimittel **dadurch gekennzeichnet, dass** es aus einer Zubereitung nach einem der Ansprüche 1-13 besteht.

17. Verfahren zur Herstellung der Zubereitung nach einem der Ansprüche 1-13, umfassend die Schritte:
(a) Bereitstellen einer Kreatin-Komponente,
(b) Einarbeiten mindestens eines Puffersystems, und
(c) ggf. Hinzufügen von zusätzlichen physiologisch aktiven Verbindungen sowie deren Derivaten und/oder Mischungen, von einem Natriumsalz, und/oder von α-Liponsäure.

## Claims

1. Preparation comprising a creatine component constituting 30-80 wt.% relative to the total weight of the preparation and a buffer system, the buffer system consisting of at least one sodium salt of an organic acid and being present in a quantity of 0.1 to 90.0 wt.%, relative to the total weight of the preparation, and the preparation having a pH value of 2.5 to 7.9, the molar ratio of creatine component to sodium ions being 1:1-10.

2. Preparation according to claim 1, **characterised in that** the organic acid is selected from malic acid, ascorbic acid, succinic acid, pyruvic acid, fumaric acid, adipic acid, aspartic acid, gluconic acid, α-ketoglutaric acid, α-lipoic acid, tartaric acid, oxalic acid, pyroglutamic acid, 3-nicotinic acid, citric acid, maleic acid, acetic acid, formic acid, 2-hydroxybenzoic acid, sorbic acid and isoascorbic acid.

3. Preparation according to either of the preceding claims, **characterised in that** the buffer system is trisodium citrate, disodium citrate and/or monosodium citrate.

4. Preparation according to any one of the preceding claims, **characterised in that** the buffer system is trisodium citrate and disodium citrate.

5. Preparation according to any one of the preceding claims, **characterised in that** the creatine component is creatine, creatine monohydrate, guanidinoacetic acid, creatine esters, creatinol or creatinol O-phosphate or a mixture of at least two of these compounds.

6. Preparation according to any one of the preceding claims, **characterised in that** the creatine component is at least one salt, at least one addition compound and/or at least one complex compound of the creatine component.

7. Preparation according to claim 5, **characterised in that** the at least one salt, the at least one addition compound and/or the at least one complex compound of the creatine component is formed with malic acid, ascorbic acid, succinic acid, pyruvic acid, fumaric acid, aspartic acid, adipic acid, gluconic acid, α-ketoglutaric acid, tartaric acid, α-lipoic acid, oxalic acid, pyroglutamic acid, 3-nicotinic acid, maleic acid, sulfuric acid, acetic acid, formic acid, phosphoric acid, hydrochloric acid, 2-hydroxybenzoic acid, L-carnitine, acetyl-L-carnitine, taurine, betaine, choline and/or methionine.

8. Preparation according to any one of the preceding claims, **characterised in that** the creatine component is provided for administration in a daily dose of 5 mg to 400 mg per kg of body weight.

9. Preparation according to any one of the preceding claims, **characterised in that** the preparation additionally contains physiologically active compounds selected from the group of carbohydrates, saturated or unsaturated fatty acids, saturated or unsaturated triglycerides, amino acids, amino acid derivatives such as taurine, carnitine, glutathione, or proteins such as for example gelatin, vitamins, vitaminoids such as for example ubiquinones, α-lipoic acid, inositol, phospholipids, minerals and trace elements and mixtures thereof.

10. Preparation according to any one of the preceding claims, **characterised in that** the preparation additionally contains a sodium salt or a mixture thereof and/or α-lipoic acid.

11. Preparation according to claim 10, **characterised in that** the sodium salt or the mixture thereof is selected from sodium chloride, sodium sulfate, sodium acetate, sodium adipate, sodium citrate, sodium gluconate, sodium ascorbate, sodium pantothenate, sodium tartrate and sodium lactate.

12. Preparation according to either claim 10 or claim 11, **characterised in that** the sodium salt is present in a quantity of 0.1 to 75.0 wt.%, relative to the total weight of the preparation.

13. Preparation according to any one of the preceding claims, **characterised in that** the preparation is in the form of a powder, granular product, pastille, capsule, tablet, effervescent tablet, solution, syrup and/or gel product.

14. Use of the preparation according to any one of the preceding claims as a nutritional supplement, functional foodstuff, feedstuff additive and/or as an additive for cosmetic and/or dermatological formulations, in particular for topical application.

15. Use according to claim 14, **characterised in that** the cosmetic and/or dermatological formulation is a cream, a lotion, a spray, a mousse, an aqueous or aqueous-ethanolic solution, an impregnation medium for wipes, an anhydrous or hydrous stick or a microemulsion.

16. Medicament **characterised in that** it consists of a preparation according to any one of claims 1-13.

17. Method for producing the preparation according to any one of claims 1-13 comprising the steps:
(a) provision of a creatine component,
(b) incorporation of at least one buffer system, and
(c) optional addition of additional physiologically active compounds and the derivatives and/or mixtures thereof, of a sodium salt, and/or α-lipoic acid.

## Revendications

1. Préparation comprenant un composant de créatine, dans une proportion de 30 à 80 % en poids par rapport au poids total de la préparation et un système tampon, lequel est constitué d'au moins un sel de sodium d'un acide organique et est présent dans une proportion comprise entre 0,1 et 90,0 % en poids par rapport au poids total de la préparation, celle-ci présentant une valeur de pH comprise entre 2,5 et 7,9, le rapport molaire entre le composant de créatine et les ions sodium étant compris entre 1 : 1 à 10.

2. Préparation selon la revendication 1, **caractérisée en ce que** l'acide organique est choisi parmi les acides malique, ascorbique, succinique, pyruvique, fumarique, adipique, aspartique, gluconique, α-cétoglutarique, α-lipoïque, tartrique, oxalique, pyroglutamique, 3-nicotinique, citrique, maléique, acétique, formique, 2-hydroxybenzoïque, sorbique et isoascorbique.

3. Préparation selon l'une des revendications précédentes, caractérisée en que le système tampon est du citrate trisodique, du citrate disodique et/ou du citrate monosodique.

4. Préparation selon l'une des revendications précédentes, caractérisée en que le système tampon est du citrate trisodique et du citrate disodique.

5. Préparation selon l'une des revendications précédentes, caractérisée en que le composant de créatine est la créatine, le monohydrate de créatine, l'acide guanidinoacétique, l'ester de créatine, le créatinol ou le O-phosphate de créatinol ou un mélange d'au moins deux de ces composés.

6. Préparation selon l'une des revendications précédentes, caractérisée en que le composant de créatine est au moins un sel, au moins un composé d'addition et/ou au moins un composé complexe du composant de créatine.

7. Préparation selon la revendication 5, **caractérisée en ce que** le au moins un sel, le au moins un composé d'addition et/ou le au moins un composé complexe du composant de créatine est formé avec les acides malique, ascorbique, succinique, pyruvique, fumarique, aspartique, adipique, gluconique, α-cétoglutarique, tartrique, α-lipoïque, oxalique, pyroglutamique, 3-nicotinique, maléique, sulfurique, acétique, formique, phosphorique, chlorhydrique ou 2-hydroxybenzoïque, la L-carnitine, l'acétyl-L-carnitine, la taurine, la bétaïne, la choline et/ou la méthionine.

8. Préparation selon l'une des revendications précédentes, caractérisée en que le composant de créatine est destiné à être administré à des doses journalières comprises entre 5 mg et 400 mg par kilo de poids corporel.

9. Préparation selon l'une des revendications précédentes, caractérisée en qu'elle contient en plus des composés exerçant une activité physiologique, choisis parmi le groupe des hydrates de carbone, des acides gras saturés ou insaturés, des triglycérides saturés ou insaturés, des acides aminés, des dérivés d'acides aminés tels que la taurine, la carnitine, le glutathion ou des protéines telles que la gélatine, des vitamines, des vitaminoïdes tels que la ubichinone, de l'acide α-lipoïque, de l'inositol, des phospholipides, des minéraux et des oligoéléments, ainsi que des mélanges de ceux-ci.

10. Préparation selon l'une des revendications précédentes, caractérisée en qu'elle contient en plus un sel de sodium ou un mélange de celui-ci et/ou d'acide α-lipoïque.

11. Préparation selon la revendication 10, **caractérisée en ce que** le sel de sodium ou le mélange de celui-ci est choisi parmi le chlorure de sodium, le sulfate de sodium, l'acétate de sodium, l'adipate de sodium, le citrate de sodium, le gluconate de sodium, l'ascorbate de sodium, le pantothénate de sodium, le tartrate de sodium et le lactate de sodium.

12. Préparation selon l'une des revendications 10 ou 11, **caractérisée en ce que** le sel de sodium est présent dans une proportion comprise entre 0,1 et 75,0 % en poids par rapport au poids total de la préparation.

13. Préparation selon l'une des revendications précédentes, caractérisée en qu'elle est présente sous forme de poudre, de granulé, de pastille, de capsule, de comprimé, de comprimé effervescent, de solution, de jus et/ou de produit en gelée.

14. Utilisation de la préparation selon l'une des revendications précédentes comme complément alimentaire, aliment fonctionnel, additif de fourrage et/ou additif pour des formulations cosmétiques et/ou dermatologiques, destinées en particulier à l'application topique.

15. Utilisation selon la revendication 14, **caractérisée en ce que** la formulation cosmétique et/ou dermatologique est une crème, une lotion, un aérosol, une mousse, une solution aqueuse ou aqueuse-éthanolique, un milieu d'imprégnation pour des tissus, un bâtonnet anhydre ou contenant de l'eau ou une microémulsion.

16. Médicament **caractérisé en ce qu'**il est constitué d'une préparation selon l'une des revendications 1 à 13.

17. Procédé pour la fabrication de la préparation selon l'une des revendications 1 à 13, comprenant les étapes suivantes:
a) préparation d'un composant de créatine,
b) incorporation d'au moins un système tampon et
c) le cas échéant, ajout de composés supplémentaires exerçant une activité physiologique, ainsi que de dérivés et/ou de mélanges de ceux-ci, d'un sel de sodium et/ou d'acide α-lipoïque.
